# EUROPEAN PATENT SPECIFICATION

(11) **EP 4 265 195 B1**
(45) Date of publication and mention of the grant of the patent: **16.04.2025**
(21) Application number: 21906158.7
(22) Date of filing: 27.10.2021
(51) Int. Cl.: A61B 8/08

(54) **ULTRASONIC DIAGNOSTIC APPARATUS AND CONTROL METHOD FOR ULTRASONIC DIAGNOSTIC APPARATUS**
ULTRASCHALLDIAGNOSEVORRICHTUNG UND STEUERUNGSVERFAHREN FÜR DIE ULTRASCHALLDIAGNOSEVORRICHTUNG
APPAREIL DE DIAGNOSTIC À ULTRASONS ET PROCÉDÉ DE COMMANDE D'UN APPAREIL DE DIAGNOSTIC À ULTRASONS

(30) Priority: 17.12.2020 JP 2020209315
(43) Date of publication of application: 25.10.2023
(73) Proprietor: FUJIFILM Corporation, Tokyo 106-8620 (JP)
(72) Inventor: EBATA Tetsurou, Tokyo 106-8620 (JP)
(74) Representative: Dehns Germany Partnerschaft mbB
(86) International application number: PCT/JP2021/039685
(87) International publication number: WO 2022/130794

(56) References cited:
- WO-A1-2020/044770
- WO-A1-2020/044770
- CN-A- 111 134 727
- JP-A- 2018 171 177
- JP-A- 2019 181 183
- JP-A- 2020 114 282
- JP-A- 2020 114 282
- US-A1- 2006 241 463

## Description

### BACKGROUND OF THE INVENTION

### 1. Field of the Invention

The present invention relates to an ultrasound diagnostic apparatus, and a control method of the ultrasound diagnostic apparatus which have a function of determining a type (an artery or a vein) of a blood vessel.

### 2. Description of the Related Art

For example, in a case of puncturing a blood vessel of a subject, in order to determine a puncture position and the thickness of a puncture needle, an ultrasound image is observed by using an ultrasound diagnostic apparatus to check the type, size, position, depth, and traveling of the blood vessel, and the presence or absence of an obstacle such as nerves. In particular, since mistakes in puncturing arteries and veins directly lead to serious medical accidents, it is important to determine the type of the blood vessel, that is, whether the blood vessel is an artery or a vein.

Here, there are JP2008-545502A and JP2018-202147A as the documents in the related art that are references for the present invention.

JP2008-545502A discloses that arteries and veins are distinguished by applying a color Doppler technique to detect the direction and pulsation of the blood flow in the arteries and the veins. JP2008-545502A discloses that arteries and veins are distinguished by using the fact that the veins collapse in a case where a mechanical pressure is applied to a tissue with blood vessels, or by measuring and analyzing the absorption of two wavelengths on the basis of the oxygen content in the blood.

JP2018-202147A discloses that arteries and veins are decided by analyzing image data using anatomical prior information indicating typical positions of the arteries and the veins. WO2020044770 is directed to an ultrasonic diagnostic device and an ultrasonic diagnostic device control method that make it possible to perform compression testing at appropriate locations, regardless of the proficiency of a user. The device comprises an ultrasonic probe; an image acquisition part that transmits an ultrasonic beam toward the subject from the ultrasonic probe and acquires an ultrasonic image; a vein detection part that detects popliteal veins that are included in the ultrasonic image acquired by the image acquisition part; and an operation guide part that, when compression testing of the popliteal vein is performed, guides operation of the ultrasonic probe on the basis of the number of popliteal veins detected by the vein detection part so as to make a user position the ultrasonic probe in a position at which only one popliteal vein is included in the ultrasonic image acquired by the image acquisition part.

### SUMMARY OF THE INVENTION

As in JP2018-202147A, in a case where the type of the blood vessel is determined only on the basis of the image data, erroneous determination may be performed. As described above, since erroneous determination of the type of the blood vessel directly leads to medical accidents, it is desirable to avoid that the type of the blood vessel is determined only on the basis of the image data.

On the other hand, as in JP2008-545502A, in a case where the type of the blood vessel is determined on the basis of data other than the image data, additional work is always required to determine the type of the blood vessel, which causes the deterioration of the usability.

An object of the present invention is to provide an ultrasound diagnostic apparatus and a control method of the ultrasound diagnostic apparatus which can prevent the type of the blood vessel from being erroneously determined, and determine the type of the blood vessel without the deterioration of the usability.

**In** order to achieve the object, an aspect of the present invention provides an ultrasound diagnostic apparatus including an ultrasound probe; an image generation unit that generates an ultrasound image from a reception signal obtained by performing transmission and reception of an ultrasound beam with respect to a subject using the ultrasound probe; a probability calculation unit that calculates each of a probability that a blood vessel in the ultrasound image is an artery and a probability that the blood vessel is a vein by analyzing the ultrasound image; a blood vessel determination unit that determines whether the blood vessel is the artery or the vein or whether or not confirmation processing for confirming whether the blood vessel is the artery or the vein is required on the basis of the probability that the blood vessel is the artery and the probability that the blood vessel is the vein; and a blood vessel confirmation unit that executes the confirmation processing in a case where it is determined that the confirmation processing is required, and confirms whether the blood vessel is the artery or the vein on the basis of an execution result of the confirmation processing.

Here, it is preferable that the probability calculation unit detects a blood vessel region with a predetermined shape, including a blood vessel in the ultrasound image, in a case of calculating a probability of being the artery and a probability of being the vein.

Further, it is preferable that the blood vessel region is an artery region including the artery or a vein region including the vein, and the probability calculation unit detects the artery region including the blood vessel in the ultrasound image, calculates a probability that the blood vessel in the artery region is the artery, detects the vein region including the same blood vessel, and calculates a probability that the same blood vessel in the vein region is the vein.

It is preferable that the probability calculation unit detects a single blood vessel region with the predetermined shape, including a blood vessel in the ultrasound image, and further calculates a probability that the blood vessel in the single blood vessel region is the artery and a probability that the blood vessel in the single blood vessel region is the vein.

It is preferable that, in a first case in which a probability of being one of the artery and the vein is equal to or greater than a first threshold value and a probability of being the other of the artery and the vein is less than a second threshold value smaller than the first threshold value, the blood vessel determination unit determines that the blood vessel is one of the artery and the vein, and in a second case other than the first case, the blood vessel determination unit determines that the confirmation processing is required.

It is preferable that the probability calculation unit calculates, for each pixel of the ultrasound image, each of a probability that the pixel is a pixel of the artery, a probability that the pixel is a pixel of the vein, and a probability that the pixel is a pixel of background other than the blood vessel.

It is preferable that the blood vessel determination unit decides, for each pixel of the ultrasound image, whether the pixel is the pixel of the artery, the pixel is the pixel of the vein, or the pixel is the pixel of the background on the basis of the probability that the pixel is the pixel of the artery, the probability that the pixel is the pixel of the vein, and the probability that the pixel is the pixel of the background, decides a blood vessel region including the blood vessel on the basis of the pixel of the artery and the pixel of the vein, determines that the blood vessel is one of the artery and the vein in a case where a ratio of one of an area of the pixel of the artery and an area of the pixel of the vein to an area of the blood vessel in the blood vessel region is equal to or greater than a third threshold value, and determines that the confirmation processing is required in a case where the ratio is less than the third threshold value.

It is preferable that the blood vessel confirmation unit prompts a user to press an examination location of the subject with the ultrasound probe, calculates an aspect ratio of a cross section of the blood vessel on the basis of an ultrasound image generated while the examination location is pressed with the ultrasound probe, and confirms whether the blood vessel is the artery or the vein on the basis of the aspect ratio of the cross section of the blood vessel.

It is preferable that the ultrasound probe has a pressure sensor, and the blood vessel confirmation unit determines whether or not the examination location is pressed on the basis of a pressure detected by the pressure sensor.

It is preferable that the blood vessel confirmation unit detects motion in the examination location by analyzing the ultrasound images of a plurality of frames, and determines whether or not the examination location is pressed on the basis of the motion in the examination location.

It is preferable that the blood vessel confirmation unit determines presence or absence of a change in the aspect ratio of the cross section of the blood vessel only for a predetermined period of time after prompting the user to press the examination location with the ultrasound probe, and determines whether or not the examination location is pressed on the basis of the presence or absence of the change in the aspect ratio of the blood vessel in the predetermined period of time.

It is preferable that the blood vessel confirmation unit prompts a user to incline the ultrasound probe at an examination location of the subject, and confirms whether the blood vessel is the artery or the vein on the basis of a direction of a blood flow in an ultrasound image generated while the ultrasound probe is inclined at the examination location in a color Doppler mode.

It is preferable that the blood vessel confirmation unit searches for an ultrasound image of a frame in which a probability that the same blood vessel is the artery or a probability that the same blood vessel is the vein is equal to or greater than a fourth threshold value among ultrasound images of past frames, and confirms whether the blood vessel is the artery or the vein on the basis of the probability that the same blood vessel in the ultrasound image of the searched frame is the artery or the probability that the same blood vessel is the vein.

It is preferable that the blood vessel confirmation unit detects the blood vessel in ultrasound images of adjacent preceding and succeeding frames, and determines that the blood vessels are the same in a case where an overlapping ratio of the blood vessels in the ultrasound images of the preceding and succeeding frames is equal to or greater than the fourth threshold value.

It is preferable that the ultrasound diagnostic apparatus further includes a monitor; and a display control unit that superimposes a graphic including the blood vessel on the ultrasound image to be displayed on the monitor on the basis of at least one of a determination result on whether the blood vessel is the artery or the vein or a confirmation result on whether the blood vessel is the artery or the vein.

It is preferable that the display control unit displays each of the probability that the blood vessel is the artery and the probability that the blood vessel is the vein on the monitor in addition to the graphic.

It is preferable that the display control unit displays the same graphic for the same blood vessel on the monitor once whether the blood vessel is the artery or the vein has been decided.

It is preferable that the display control unit displays characters, blood vessel, on the monitor in addition to the graphic in a case where deciding whether the blood vessel is the artery or the vein is not possible.

Another aspect of the present invention provides a control method of an ultrasound diagnostic apparatus, the control method including generating an ultrasound image from a reception signal obtained by performing transmission and reception of an ultrasound beam with respect to a subject using an ultrasound probe; calculating a probability that a blood vessel in the ultrasound image is an artery and a probability that the blood vessel is a vein by analyzing the ultrasound image; determining whether the blood vessel is the artery or the vein or whether or not confirmation processing for confirming whether the blood vessel is the artery or the vein is required on the basis of the probability that the blood vessel is the artery and the probability that the blood vessel is the vein; and executing the confirmation processing in a case where it is determined that the confirmation processing is required, and confirming whether the blood vessel is the artery or the vein on the basis of an execution result of the confirmation processing.

In the present invention, the type of the blood vessel is determined on the basis of the probability that the blood vessel is an artery and the probability that the blood vessel is a vein as described above, and in a case where the type of the blood vessel cannot be determined, the confirmation processing is executed to confirm whether the blood vessel is an artery or a vein. Therefore, it is possible to prevent the type of the blood vessel from being erroneously determined, and to greatly improve the determination accuracy of the type of the blood vessel. Further, in the present invention, since the confirmation processing is executed only in a case where the type of the blood vessel cannot be determined, it is possible to greatly suppress the deterioration of the usability.

### BRIEF DESCRIPTION OF THE DRAWINGS

Fig. 1 is a block diagram of an embodiment illustrating a configuration of an ultrasound diagnostic apparatus.
Fig. 2 is a block diagram of an embodiment illustrating a configuration of a transmission and reception circuit.
Fig. 3 is a block diagram of an embodiment illustrating a configuration of an image generation unit.
Fig. 4 is a block diagram of an embodiment illustrating a configuration of a blood vessel processing unit.
Fig. 5 is a flowchart of an embodiment illustrating an operation of an ultrasound diagnostic apparatus in a case of capturing an ultrasound image.
Fig. 6 is a flowchart of an embodiment illustrating an operation of an ultrasound diagnostic apparatus in a case of determining the type of the blood vessel in an ultrasound image.
Fig. 7 is a flowchart of another embodiment illustrating an operation of an ultrasound diagnostic apparatus in a case of determining the type of the blood vessel in an ultrasound image.
Fig. 8 is a conceptual diagram of an embodiment illustrating an ultrasound image in which an enclosing line enclosing a region of a cross section of a blood vessel is superimposed and displayed.
Fig. 9 is a conceptual diagram of another embodiment illustrating an ultrasound image in which an enclosing line enclosing a region of a cross section of a blood vessel is superimposed and displayed.
Fig. 10 is a conceptual diagram of an embodiment illustrating an ultrasound image on which a graphic representing a region of a cross section of a blood vessel is superimposed and displayed.
Fig. 11 is a conceptual diagram of another embodiment illustrating an ultrasound image on which a graphic representing a region of a cross section of a blood vessel is superimposed and displayed.
Fig. 12 is a conceptual diagram of an embodiment illustrating a message prompting a user to press an examination location of a subject with an ultrasound probe.
Fig. 13A is a conceptual diagram of an embodiment illustrating an ultrasound image in a state where an examination location is not pressed by an ultrasound probe.
Fig. 13B is a conceptual diagram of an embodiment illustrating an ultrasound image in a state where an examination location is pressed by an ultrasound probe.
Fig. 14 is a conceptual diagram of an embodiment illustrating a message prompting a user to incline an ultrasound probe at an examination location of a subject.

### DESCRIPTION OF THE PREFERRED EMBODIMENTS

Hereinafter, an ultrasound diagnostic apparatus and a control method of the ultrasound diagnostic apparatus according to the present invention will be described in detail on the basis of preferred embodiments illustrated in the accompanying drawings.

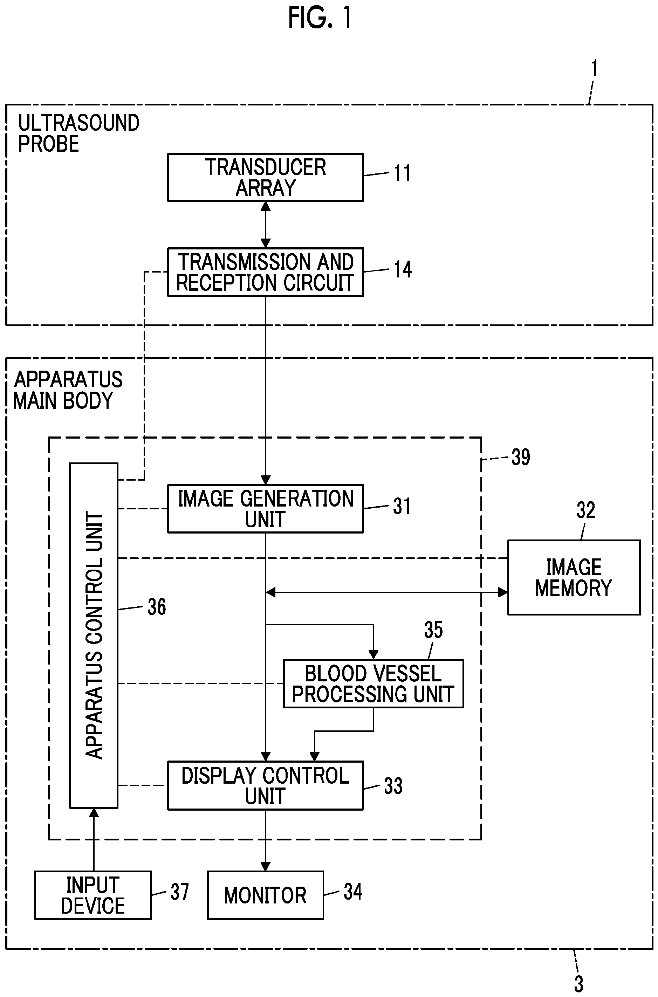

Fig. 1 is a block diagram of an embodiment illustrating a configuration of an ultrasound diagnostic apparatus of the present invention. An ultrasound diagnostic apparatus 20 illustrated in Fig. 1 includes an ultrasound probe 1, and an apparatus main body 3 connected to the ultrasound probe 1.

The ultrasound probe 1 scans an examination location of a subject using an ultrasound beam, and outputs a sound ray signal corresponding to an ultrasound image of the examination location. As illustrated in Fig. 1, the ultrasound probe 1 includes a transducer array 11, and a transmission and reception circuit 14. The transducer array 11 and the transmission and reception circuit 14 are bidirectionally connected to each other. Further, an apparatus control unit 36, which will be described later, of the apparatus main body 3 is connected to the transmission and reception circuit 14.

The transducer array 11 has a plurality of ultrasonic transducers arranged in a one-dimensional or two-dimensional manner. According to a drive signal supplied from the transmission and reception circuit 14, each of the transducers transmits an ultrasonic wave and receives a reflected wave from the subject to output an analog reception signal.

For example, each transducer is formed by using an element in which electrodes are formed at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

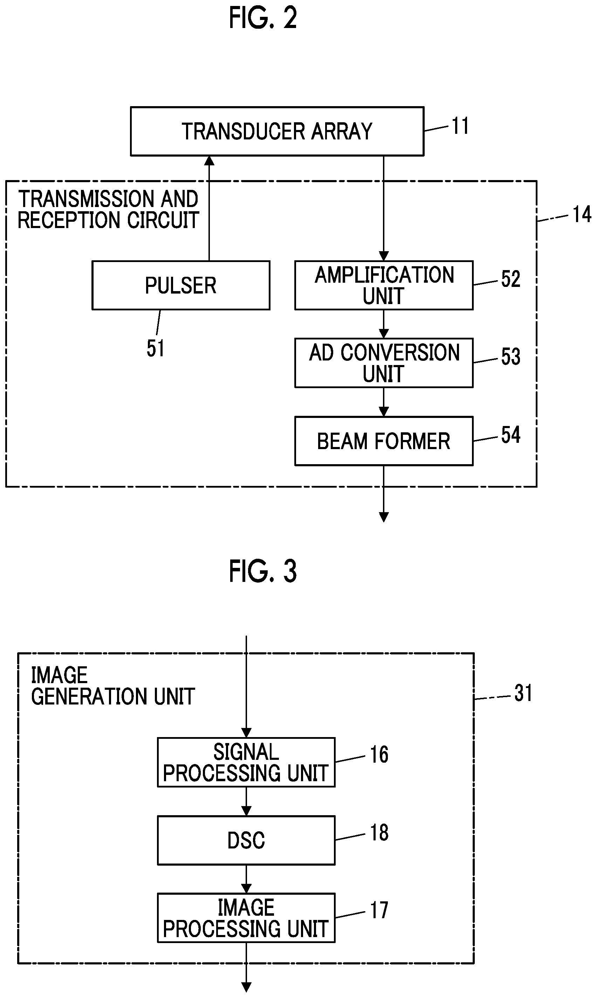

The transmission and reception circuit 14 causes the transducer array 11 to transmit the ultrasonic wave, and performs reception focusing processing on the reception signal output from the transducer array 11 that has received the ultrasound echo to generate a sound ray signal, under the control of the apparatus control unit 36. As illustrated in Fig. 2, the transmission and reception circuit 14 has a pulser 51 connected to the transducer array 11, and an amplification unit 52, an analog to digital (AD) conversion unit 53, and a beam former 54 that are sequentially connected in series from the transducer array 11.

The pulser 51 includes, for example, a plurality of pulse generators, and the pulser 51 adjusts the amount of delay of each drive signal so that ultrasonic waves transmitted from the plurality of transducers of the transducer array 11 form an ultrasound beam on the basis of a transmission delay pattern selected by the apparatus control unit 36, and supplies the obtained signals to the plurality of transducers. Thus, in a case where a pulsed or continuous-wave voltage is applied to the electrodes of the transducers of the transducer array 11, the piezoelectric body expands and contracts to generate pulsed or continuous-wave ultrasonic waves from each transducer. From the combined wave of these ultrasonic waves, an ultrasound beam is formed.

The transmitted ultrasound beam is reflected by a target, for example, a site of the subject, and propagates toward the transducer array 11 of the ultrasound probe 1. Each transducer constituting the transducer array 11 expands and contracts by receiving the ultrasound echo propagating toward the transducer array 11 in this manner, to generate the reception signal that is an electric signal, and outputs the reception signal to the amplification unit 52.

The amplification unit 52 amplifies the signals input from each transducer constituting the transducer array 11, and transmits the amplified signals to the AD conversion unit 53. The AD conversion unit 53 converts the analog signal transmitted from the amplification unit 52 into digital reception data, and outputs the reception data to the beam former 54.

The beam former 54 performs so-called reception focusing processing in which addition is performed by giving delays to respective pieces of the reception data converted by the AD conversion unit 53 according to a sound speed distribution or a sound speed set on the basis of a reception delay pattern selected by the apparatus control unit 36. Through the reception focusing processing, a sound ray signal in which each piece of the reception data converted by the AD conversion unit 53 is phased and added and the focus of the ultrasound echo is narrowed is generated.

Next, the apparatus main body 3 generates an ultrasound image of the examination location of the subject on the basis of the sound ray signal generated by the ultrasound probe 1, and displays the ultrasound image of the examination location of the subject. As illustrated in Fig. 1, the apparatus main body 3 includes an image generation unit 31, an image memory 32, a blood vessel processing unit 35, a display control unit 33, a monitor (display unit) 34, an input device 37, and the apparatus control unit 36.

The image generation unit 31 is connected to the transmission and reception circuit 14, and the display control unit 33 and the monitor 34 are sequentially connected in series to the image generation unit 31. Each of the image memory 32 and the blood vessel processing unit 35 is connected to the image generation unit 31, and the display control unit 33 is connected to the image memory 32 and the blood vessel processing unit 35. The apparatus control unit 36 is connected to the transmission and reception circuit 14, the image generation unit 31, the display control unit 33, the image memory 32, and the blood vessel processing unit 35, and the input device 37 is connected to the apparatus control unit 36.

The image generation unit 31 generates the ultrasound image (ultrasound image signal) of the examination location of the subject, from the reception signal obtained by performing transmission and reception of the ultrasound beams with respect to the examination location of the subject using the ultrasound probe 1 (more precisely, transducer array 11), in other words, from the sound ray signal generated from the reception signal by the transmission and reception circuit 14, under the control of the apparatus control unit 36. As illustrated in Fig. 3, the image generation unit 31 has a configuration in which a signal processing unit 16, a digital scan converter (DSC) 18, and an image processing unit 17 are sequentially connected in series.

The signal processing unit 16 generates image information data corresponding to the ultrasound image on the basis of the sound ray signal generated by the transmission and reception circuit 14. More specifically, the signal processing unit 16 generates the image information data representing tomographic image information regarding tissues inside the subject, by performing envelope detection processing after signal processing, for example, correcting the attenuation of the sound ray signal generated by the beam former 54 of the transmission and reception circuit 14, which is caused by the propagation distance according to the depth of the reflection position of the ultrasonic wave.

The DSC 18 raster-converts the image information data generated by the signal processing unit 16 into an image signal according to a normal television signal scanning method.

The image processing unit 17 performs various kinds of image processing such as brightness correction, gradation correction, sharpness correction, image size correction, refresh rate correction, scanning frequency correction, and color correction according to a display format of the monitor 34, on the image signal input from the DSC 18 to generate the ultrasound image (ultrasound image signal), and then outputs the ultrasound image on which the image processing has been performed, to the image memory 32, the blood vessel processing unit 35, and the display control unit 33.

The image memory 32 is a memory that stores ultrasound images (ultrasound image signal) of the series of a plurality of frames, which are generated for each examination location by the image generation unit 31, under the control of the apparatus control unit 36. Here, as the image memory 32, recording media such as a flash memory, a hard disk drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), an external server, or the like can be used.

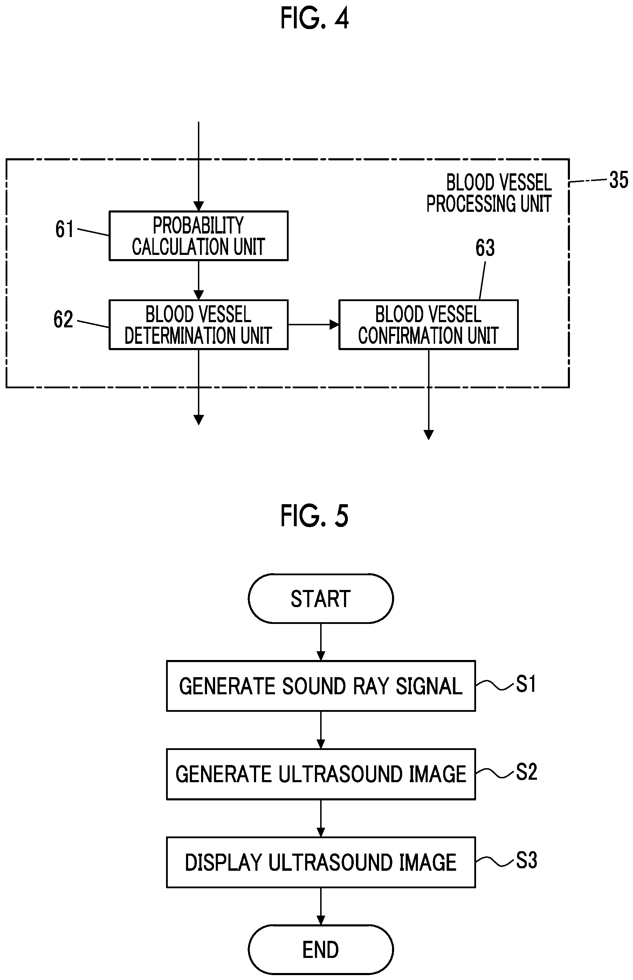

The blood vessel processing unit 35 performs various kinds of processing for determining the type of the blood vessel in the ultrasound image of the examination location of the subject, that is, whether the blood vessel is an artery or a vein, under the control of the apparatus control unit 36. As illustrated in Fig. 4, the blood vessel processing unit 35 includes a probability calculation unit 61, a blood vessel determination unit 62, and a blood vessel confirmation unit 63. The blood vessel determination unit 62 and the blood vessel confirmation unit 63 are connected in series to the probability calculation unit 61.

The probability calculation unit 61 calculates each of a probability that the blood vessel in the ultrasound image is an artery and a probability that the blood vessel in the ultrasound image is a vein by analyzing the ultrasound image of the examination location of the subject.

Although not particularly limited, the probability calculation unit 61 can calculate the probability that the blood vessel is an artery and the probability that the blood vessel is a vein by analyzing the ultrasound image using at least one of a machine learning model trained using a machine learning technique such as deep learning, template matching, and an image analysis technique using feature amounts such as Adaptive Boosting (Adaboost), support vector machines (SVM) or scale-invariant feature transform (SIFT).

The blood vessel determination unit 62 determines whether the blood vessel is an artery or a vein, or whether or not additional confirmation processing for confirming whether the blood vessel is an artery or a vein is required, on the basis of the probability that the blood vessel is an artery and the probability that the blood vessel is a vein. In other words, the blood vessel determination unit 62 determines whether the type of the blood vessel can be decided or cannot be decided, only on the basis of the probability that the blood vessel is an artery or the probability that the blood vessel is a vein.

The confirmation processing will be specifically described later.

In a case where it is determined that the confirmation processing is required by the blood vessel determination unit 62, the blood vessel confirmation unit 63 executes the confirmation processing, and confirms that the blood vessel in the ultrasound image is an artery or a vein on the basis of an execution result of the confirmation processing.

The display control unit 33 displays various kinds of information on the monitor 34 under the control of the apparatus control unit 36. For example, the display control unit 33 performs predetermined processing on the ultrasound image held in the image memory 32, and displays the processed ultrasound image on the monitor 34. Further, the display control unit 33 displays a determination result on whether the blood vessel is an artery or a vein, a confirmation result on whether the blood vessel is an artery or a vein, and the like on the monitor 34.

The monitor 34 displays various kinds of information described above under the control of the display control unit 33. Examples of the monitor 34 include a display device such as a liquid crystal display (LCD), and an organic electroluminescence (EL) display.

The input device 37 receives various instructions input from the user (examiner) of the ultrasound diagnostic apparatus. Although not particularly limited, the input device 37 includes various buttons, and a touch panel or the like through which various instructions are input by the user performing a touch operation.

The apparatus control unit 36 controls the ultrasound probe 1 and each unit of the apparatus main body 3 on the basis of a program stored in advance and an instruction or the like of the user input from the input device 37.

The image generation unit 31, the blood vessel processing unit 35, the display control unit 33, and the apparatus control unit 36 constitute a terminal-side processor 39.

Next, the operation of the ultrasound diagnostic apparatus in a case where the ultrasound image is captured will be described with reference to the flowchart of Fig. 5.

First, in a state where the ultrasound probe 1 is in contact with the examination location of the subject, under the control of the apparatus control unit 36, the transmission of the ultrasonic waves is started by the transmission and reception circuit 14, and the sound ray signal is generated (Step S1).

That is, the ultrasound beams are transmitted to the examination location of the subject from a plurality of transducers of the transducer array 11 according to the drive signals from the pulser 51.

Ultrasound echoes from the examination location based on the ultrasound beams transmitted from the pulser 51 are received by each transducer of the transducer array 11, and the reception signal as an analog signal is output from each transducer of the transducer array 11, which has received the ultrasound echo.

The reception signal output from each transducer of the transducer array 11 is amplified by the amplification unit 52, and is subjected to AD conversion by the AD conversion unit 53, and thereby the reception data is acquired.

By performing the reception focusing processing on the reception data by the beam former 54, the sound ray signal is generated.

Next, under the control of the apparatus control unit 36, the ultrasound image (ultrasound image signal) of the examination location of the subject is generated by the image generation unit 31 on the basis of the sound ray signal generated by the beam former 54 of the transmission and reception circuit 14 (Step S2).

That is, the sound ray signal generated by the beam former 54 is subjected to various kinds of signal processing by the signal processing unit 16, and the image information data representing tomographic image information regarding tissues inside the subject is generated.

The image information data generated by the signal processing unit 16 is raster-converted by the DSC 18, and is further subjected to various kinds of image processing by the image processing unit 17, and thus the ultrasound image (ultrasound image signal) is generated.

The ultrasound image generated by the image processing unit 17 is held in the image memory 32.

Next, under the control of the apparatus control unit 36, predetermined processing is performed on the ultrasound image held in the image memory 32 by the display control unit 33, and the processed ultrasound image is displayed on the monitor 34 (Step S3).

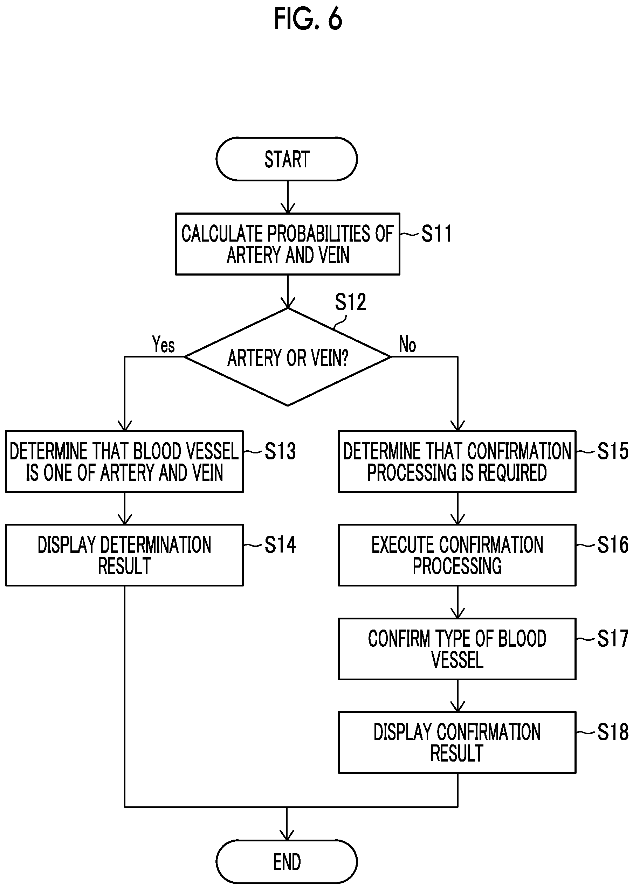

Next, the operation of the ultrasound diagnostic apparatus in a case of determining the type of the blood vessel in the ultrasound image will be described. First, with reference to the flowchart of Fig. 6, in a case of calculating the probability of being an artery and the probability of being a vein, the operation of the ultrasound diagnostic apparatus in a case of detecting a blood vessel region (artery region, vein region) with a predetermined shape such as a rectangular shape, a circular shape, or a polygonal shape, including the blood vessel in the ultrasound image, and determining the type of the blood vessel in the ultrasound image will be described.

First, by the probability calculation unit 61, the blood vessel region in the ultrasound image is detected, and the probability that the blood vessel in the blood vessel region is an artery and the probability that the blood vessel in the blood vessel region is a vein are calculated (Step S11).

The blood vessel region is one of an artery region including an artery or a vein region including a vein.

For example, the probability calculation unit 61 detects the artery region including the blood vessel in the ultrasound image, and calculates the probability that the blood vessel in the artery region is an artery, by using the machine learning model. Further, the probability calculation unit 61 detects the vein region including the same blood vessel, and calculates the probability that the blood vessel in the vein region is a vein. That is, the probability calculation unit 61 performs the calculation of the probability that the blood vessel is an artery and the calculation of the probability that the blood vessel is a vein, for the same single blood vessel in two steps.

Here, the machine learning model of the artery is a trained model in which learning ultrasound images and ground truth data of the artery region including the blood vessel in the learning ultrasound image and the probability that the blood vessel in the artery region is an artery are used as teacher data, and which has been trained for a relationship between the learning ultrasound image and the ground truth data of the artery region and the probability that the blood vessel in the artery region is an artery in the learning ultrasound image, for a plurality of pieces of the teacher data.

In this case, in the machine learning model of the artery, the ultrasound image of the examination location of the subject at the time of examination is input, and an estimation result of estimating the artery region in the ultrasound image and the probability that the blood vessel in the artery region is an artery is output.

Then, by the probability calculation unit 61, the artery region in the ultrasound image is detected, and the probability that the blood vessel in the artery region is an artery is calculated on the basis of the estimation result by the machine learning model of the artery.

Further, the machine learning model of the vein is a trained model in which learning ultrasound images and ground truth data of the vein region in the learning ultrasound image and the probability of being a vein are used as teacher data, and which has been trained for a relationship between the learning ultrasound image and the ground truth data of the vein region and the probability of being a vein in the learning ultrasound image, for a plurality of pieces of the teacher data.

In this case, in the machine learning model of the vein, the ultrasound image of the examination location of the subject at the time of examination is input, and an estimation result of estimating the vein region in the ultrasound image and the probability of being a vein is output.

Then, by the probability calculation unit 61, the vein region in the ultrasound image is detected, and the probability that the blood vessel in the vein region is a vein is calculated on the basis of the estimation result by the machine learning model of the vein.

Similarly, the probability calculation unit 61 may detect a single blood vessel region with a predetermined shape, including the blood vessel in the ultrasound image, and calculate the probability that the blood vessel in the single blood vessel region is an artery and the probability that the blood vessel in the single blood vessel region is a vein, by using the machine learning model. The single blood vessel region means a region including a single blood vessel without distinguishing the artery region or the vein region. That is, the probability calculation unit 61 performs the calculation of the probability that the blood vessel is an artery and the probability that the blood vessel is a vein at once.

Here, the machine learning model is a trained model in which learning ultrasound images and ground truth data of the single blood vessel region with a predetermined shape, including the blood vessel in the learning ultrasound image, the probability that the blood vessel in the single blood vessel region is an artery, and the probability that the blood vessel in the single blood vessel region is a vein are used as teacher data, and which has been trained for a relationship between the learning ultrasound image and the ground truth data of the single blood vessel region in the learning ultrasound image, the probability that the blood vessel in the single blood vessel region is an artery, and the probability that the blood vessel in the single blood vessel region is a vein, for a plurality of pieces of the teacher data.

In this case, in the machine learning model, the ultrasound image of the examination location of the subject at the time of examination is input, and an estimation result of estimating the single blood vessel region in the ultrasound image, the probability that the blood vessel in the single blood vessel region is an artery, and the probability that the blood vessel in the single blood vessel region is a vein is output.

Then, by the probability calculation unit 61, the single blood vessel region in the ultrasound image is detected, and the probability that the blood vessel in the single blood vessel region is an artery and the probability that the blood vessel in the single blood vessel region is a vein are calculated on the basis of the estimation result by the machine learning model.

Subsequently, by the blood vessel determination unit 62, whether the blood vessel is an artery or a vein, or whether or not the confirmation processing is required is determined on the basis of the probability that the blood vessel is an artery and the probability that the blood vessel is a vein (Step S12).

Here, in a first case in which the probability of being one of an artery and a vein is equal to or greater than a first threshold value, and the probability of being the other of an artery and a vein is less than a second threshold value smaller than the first threshold value (Yes in Step S12), it is determined that the blood vessel is one of an artery or a vein by the blood vessel determination unit 62 (Step S13).

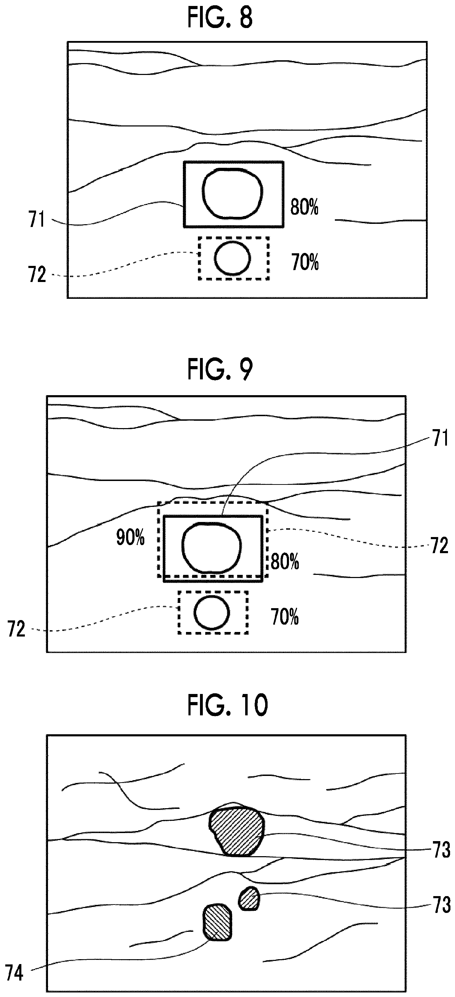

In a case where it is determined that the blood vessel is one of an artery or a vein, the graphic including the blood vessel is superimposed on the ultrasound image to be displayed on the monitor 34 on the basis of the determination result by the display control unit 33 (Step S14). For example, as illustrated in Fig. 8, as the graphic, a rectangular enclosing line surrounding the region of the cross section of the blood vessel is superimposed on the ultrasound image to be displayed on the monitor 34. Further, in addition to the enclosing line, the probability that the blood vessel is an artery and the probability that the blood vessel is a vein are respectively displayed on the monitor 34.

In the present invention, displaying the probability that the blood vessel is an artery and the probability that the blood vessel is a vein on the monitor 34 is not essential. That is, the probability that the blood vessel is an artery and the probability that the blood vessel is a vein may be displayed or may not be displayed on the monitor 34.

In the example illustrated in Fig. 8, in a central portion of the ultrasound image, a rectangular enclosing line 71 including an artery is superimposed and displayed on the ultrasound image, and in addition, characters "80%" are displayed as the probability of being an artery. Further, in a central lower portion of the ultrasound image, a rectangular enclosing line 72 including a vein is superimposed and displayed on the ultrasound image, and in addition, characters "70%" are displayed as the probability of being a vein.

As illustrated in Fig. 8, the probability that the blood vessel in the central portion of the ultrasound image is an artery is 80%, the probability that the blood vessel in the central portion of the ultrasound image is a vein is 0%, and for example, it is assumed that the first threshold value is 80% and the second threshold value is 20%. In this case, since 80% of the probability that the blood vessel is an artery is equal to or greater than 80% of the first threshold value, and 0% of the probability that the blood vessel is a vein is less than 20% of the second threshold value, the blood vessel determination unit 62 determines that the blood vessel is an artery.

In Fig. 8, the enclosing line 71 including the artery is displayed by a solid line, and the enclosing line 72 including the vein is displayed by a dotted line, but the brightness, color, line type, shape, and the like of the enclosing lines 71 and 72 are not particularly limited.

On the other hand, in a second case other than the first case described above (No in Step S12), that is, in a case other than the case in which the probability that the blood vessel is one of an artery and a vein is equal to or greater than the first threshold value, and the probability that the blood vessel is the other of an artery and a vein is less than the second threshold value smaller than the first threshold value, it is determined that the additional confirmation processing is required by the blood vessel determination unit 62 (Step S15).

For example, in the example illustrated in Fig. 9, in the blood vessel in the central portion of the ultrasound image, the rectangular enclosing line 71 representing the artery is superimposed and displayed on the ultrasound image, and in addition, characters "80%" are displayed as the probability of being an artery. Further, in the same single blood vessel, the rectangular enclosing line 72 representing the vein is superimposed and displayed on the ultrasound image, and in addition, characters "90%" are displayed as the probability of being a vein.

As illustrated in Fig. 9, the probability that the blood vessel in the central portion of the ultrasound image is an artery is 80%, the probability that the blood vessel in the central portion of the ultrasound image is a vein is 90%, and similar to the example in Fig. 8, it is assumed that the first threshold value is 80% and the second threshold value is 20%. In this case, since 80% of the probability that the blood vessel is an artery is equal to or greater than 80% of the first threshold value, but 90% of the probability that the blood vessel is a vein is not less than 20% of the second threshold value, the blood vessel determination unit 62 determines that the confirmation processing is required.

The ultrasound image illustrated in Fig. 9 is a conceptual diagram for describing a state of a case where, for the same single blood vessel, both the probability of being an artery and the probability of being a vein are not 0%, and is not displayed on the monitor 34.

In a case where it is determined that the confirmation processing is required, by the blood vessel confirmation unit 63, the confirmation processing is executed (Step S16), and the type of the blood vessel, that is, whether the blood vessel is an artery or a vein is confirmed on the basis of the execution result (Step S17).

In a case where it is confirmed whether the blood vessel is an artery or a vein, the graphic including the blood vessel is superimposed on the ultrasound image to be displayed on the monitor 34 on the basis of the confirmation result by the display control unit 33 (Step S18). For example, similar to the example illustrated in Fig. 8, as the graphic, a rectangular enclosing line surrounding the region of the cross section of the blood vessel is superimposed on the ultrasound image to be displayed on the monitor 34.

The operation described above is repeatedly performed for each frame of the ultrasound images and for each blood vessel region.

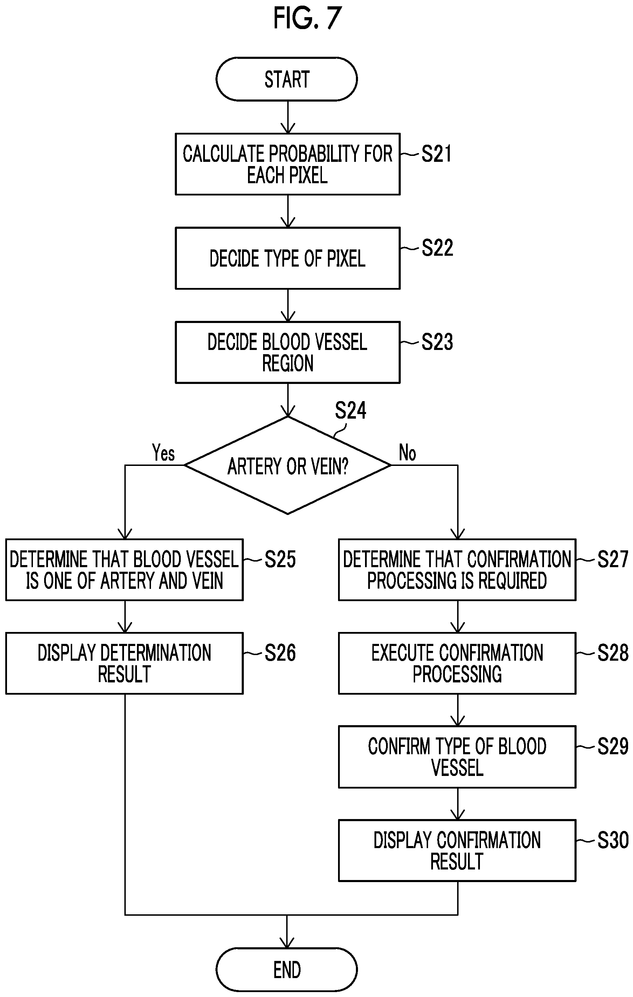

Next, with reference to the flowchart of Fig. 7, the operation of the ultrasound diagnostic apparatus in a case of deciding whether the pixel is a pixel of an artery or a pixel of a vein for each pixel in the ultrasound image, and determining the type of the blood vessel on the basis of the result will be described.

First, by the probability calculation unit 61, each of the probability that the pixel is a pixel of an artery, the probability that the pixel is a pixel of a vein, and the probability that the pixel is a pixel of the background other than the blood vessel is calculated for each pixel of the ultrasound image (Step S21). For example, the probability calculation unit 61 calculates each of the probability that the pixel is a pixel of an artery, the probability that the pixel is a pixel of a vein, and the probability that the pixel is a pixel of the background other than the blood vessel by using the machine learning model for the probability estimation.

Here, the machine learning model is a trained model in which learning ultrasound images and ground truth data on whether the pixel is a pixel of an artery, is a pixel of a vein, or is a pixel of the background for each pixel of the learning ultrasound image are used as teacher data, and which has been trained for a relationship between the learning ultrasound images and the ground truth data on whether the pixel is a pixel of an artery, is a pixel of a vein, or is a pixel of the background for each pixel of the learning ultrasound image, for a plurality of pieces of the teacher data.

In this case, in the machine learning model, the ultrasound image of the examination location of the subject at the time of examination is input, and an estimation result of estimating the probability that the pixel is a pixel of an artery, the probability that the pixel is a pixel of a vein, and the probability that the pixel is a pixel of the background is output for each pixel of the ultrasound image.

Then, by the probability calculation unit 61, for each pixel of the ultrasound image, the probability that the pixel is a pixel of an artery, the probability that the pixel is a pixel of a vein, and the probability that the pixel is a pixel of the background are calculated on the basis of the estimation result by the machine learning model.

Subsequently, by the blood vessel determination unit 62, for each pixel of the ultrasound image, the type of the pixel, that is, that the pixel is a pixel of an artery, is a pixel of a vein, or is a pixel of the background is decided on the basis of the probability that the pixel is a pixel of an artery, the probability that the pixel is a pixel of a vein, and the probability that the pixel is a pixel of the background (Step S22).

For example, the blood vessel determination unit 62 decides that the same single pixel is a type of the pixel with the highest probability. That is, in a case where the probability of being a pixel of an artery is highest, it is decided that the pixel is a pixel of an artery. Similarly, in a case where the probability of being a pixel of a vein is highest, it is decided that the pixel is a pixel of a vein, and in a case where the probability of being a pixel of the background is highest, it is decided that the pixel is a pixel of the background.

Subsequently, by the blood vessel determination unit 62, the blood vessel region including the blood vessel is decided on the basis of the pixel of the artery and the pixel of the vein (Step S23). For example, the blood vessel determination unit 62 decides that the region represented by a collection (cluster) of the pixel of the artery and the pixel of the vein is a blood vessel region.

Subsequently, by the blood vessel determination unit 62, whether the blood vessel in the blood vessel region is an artery or a vein, or whether or not the confirmation processing is required is determined on the basis of the area of the pixel of the artery and the area of the pixel of the vein with respect to the area of the blood vessel in the blood vessel region (Step S24).

Here, in a case where a ratio of one of the area of the pixel of the artery or the area of the pixel of the vein to the area of the blood vessel in the blood vessel region is equal to or greater than a third threshold value (Yes in Step S24), it is determined that the blood vessel is an artery or a vein by the blood vessel determination unit 62 (Step S25).

In a case where it is determined that the blood vessel is an artery or a vein, the graphic including the blood vessel is superimposed on the ultrasound image to be displayed on the monitor 34 on the basis of the determination result by the display control unit 33 (Step S26). For example, as illustrated in Fig. 10, a graphic representing the region of the cross section of the blood vessel is superimposed on the ultrasound image to be displayed on the monitor 34.

In the example illustrated in Fig. 10, in the central portion of the ultrasound image, a graphic 73 representing the region of the pixel of the artery is superimposed and displayed on the ultrasound image. Further, on the left side in the central lower portion of the ultrasound image, a graphic 74 representing the region of the pixel of the vein is superimposed and displayed on the ultrasound image, and in the right side thereof, the graphic 73 representing the region of the pixel of the artery is superimposed and displayed on the ultrasound image.

For example, it is assumed that the area of the blood vessel in the blood vessel region in the central portion of the ultrasound image illustrated in Fig. 10 is 100, the area of the pixel of the artery in the blood vessel region is 100, the area of the pixel of the vein is 0, and the third threshold value is 0.8. In this case, the area of the pixel of the artery, which is 100, with respect to the area of the blood vessel in the blood vessel region, which is 100, is 100/100 = 1, and is equal to or greater than 0.8 as the third threshold value, and thus, the blood vessel determination unit 62 determines that the blood vessel is an artery.

In Fig. 10, as the graphics 73 and 74, predetermined hatching is applied to the region of the pixel of the artery and the region of the pixel of the vein, but the present invention is not limited thereto, and for example, the regions may be colored in a translucent color, or may be displayed with diagonal lines. In addition to this, the enclosing line described above may be displayed, or only the enclosing line may be displayed. The same applies to the example illustrated in Fig. 8 described above.

On the other hand, in a case where a ratio of one of the area of the pixel of the artery or the area of the pixel of the vein to the area of the blood vessel in the blood vessel region is less than the third threshold value (No in Step S24), it is determined that the additional confirmation processing is required by the blood vessel determination unit 62 (Step S27).

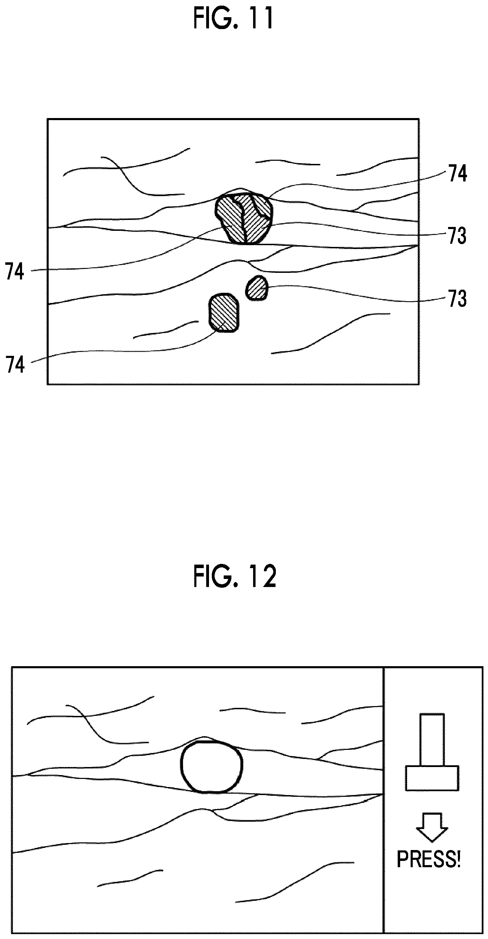

For example, in the example illustrated in Fig. 11, in the blood vessel region in the central portion of the ultrasound image, in the central portion in the left and right direction, the graphic 73 representing the region of the pixel of the artery is superimposed and displayed on the ultrasound image, and in both sides thereof, the graphics 74 representing the regions of the pixels of the vein are superimposed and displayed on the ultrasound image. That is, both the pixel of the artery and the pixel of the vein coexist in the same single blood vessel region.

For example, it is assumed that the area of the blood vessel in the blood vessel region in the central portion of the ultrasound image illustrated in Fig. 11 is 100, the area of the pixel of the artery in the blood vessel region is 40, the area of the pixel of the vein is 60, and the third threshold value is 0.8. In this case, the area of the pixel of the artery, which is 40, with respect to the area of the blood vessel in the blood vessel region, which is 100, is 40/100 = 0.4, the area of the pixel of the vein, which is 60, with respect to the area of the blood vessel in the blood vessel region, which is 100, is 60/100 = 0.6, and none of the ratios is equal to or greater than 0.8 as the third threshold value. Therefore, the blood vessel determination unit 62 determines that the confirmation processing is required.

The ultrasound image illustrated in Fig. 11 is a conceptual diagram for describing a state of a case where, for the same single blood vessel region, both the area of the pixel of the artery and the area of the pixel of the vein are not 0, and is not displayed on the monitor 34.

In a case where it is determined that the confirmation processing is required, by the blood vessel confirmation unit 63, the confirmation processing is executed (Step S28), and the type of the blood vessel, that is, whether the blood vessel is an artery or a vein is confirmed on the basis of the execution result (Step S29).

In a case where it is confirmed whether the blood vessel is an artery or a vein, the graphic including the blood vessel is superimposed on the ultrasound image to be displayed on the monitor 34 on the basis of the confirmation result by the display control unit 33 (Step S30). For example, similar to the example illustrated in Fig. 10, the graphic representing the region of the cross section of the blood vessel is superimposed on the ultrasound image to be displayed on the monitor 34.

Similarly, the operation described above is repeatedly performed for each frame of the ultrasound images and for each blood vessel region.

Next, the confirmation processing will be described. The confirmation processing is not particularly limited, but can be exemplified by a method of pressing the examination location, a method of using a color Doppler mode, and a method of using the ultrasound image of the past frame.

First, the method of pressing the examination location will be described.

In this case, the blood vessel confirmation unit 63 prompts the user to press the examination location of the subject with the ultrasound probe 1. For example, as illustrated in Fig. 12, the blood vessel confirmation unit 63 causes the display control unit 33 to display a message for prompting the user on the monitor 34. In Fig. 12, a schematic diagram representing pressing the ultrasound probe is displayed on the right portion of the ultrasound image, and on the lower side thereof, characters "press!" are displayed. Alternatively, the blood vessel confirmation unit 63 may be provided with a speaker and output sound for prompting the user from the speaker, and may prompt the user by using both the message and the sound.

Subsequently, the blood vessel confirmation unit 63 calculates the aspect ratio of the cross section of the blood vessel on the basis of the ultrasound image generated while the examination location is pressed with the ultrasound probe 1.

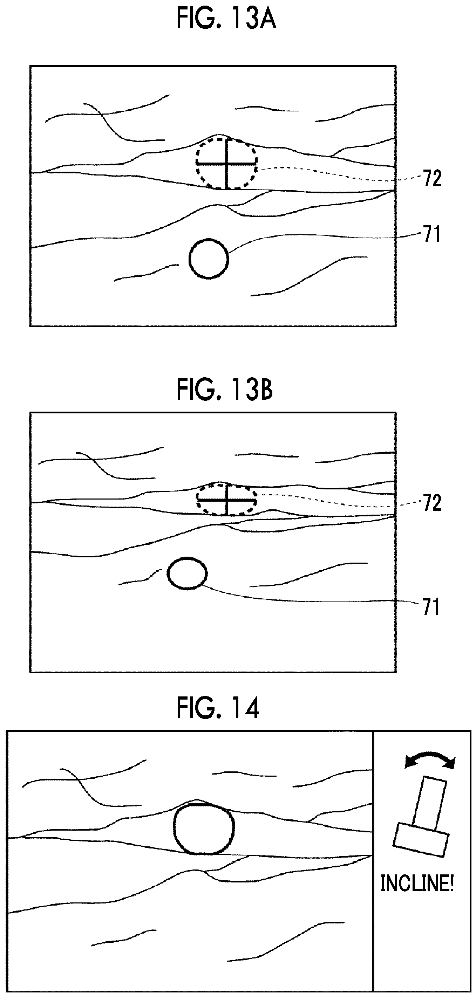

Here, in a case where a state where the examination location is not pressed as illustrated in Fig. 13A is changed to a state where the examination location is pressed as illustrated in Fig. 13B, in the examination location, the artery represented by the enclosing line 71 hardly collapses due to a high internal pressure of the artery, but the vein represented by the enclosing line 72 greatly collapses due to a low internal pressure of the vein, and thus the aspect ratio of the cross section of the vein is changed. As illustrated in Fig. 13B, for example, the length in the lateral direction/the length in the vertical direction is increased.

Then, the blood vessel confirmation unit 63 confirms whether the blood vessel is an artery or a vein on the basis of the aspect ratio of the cross section of the blood vessel. That is, the case where the aspect ratio of the cross section of the blood vessel is not changed means that the blood vessel has not collapsed, the blood vessel confirmation unit 63 confirms that the blood vessel is an artery. Conversely, the case where the aspect ratio of the cross section of the blood vessel is changed means that the blood vessel has collapsed, the blood vessel confirmation unit 63 confirms that the blood vessel is a vein.

By providing a pressure sensor that detects a pressure applied to a contact surface with the examination location of the subject, to the ultrasound probe 1, the blood vessel confirmation unit 63 can determine whether or not the examination location is pressed on the basis of the pressure detected by the pressure sensor.

The blood vessel confirmation unit 63 can detect the motion in the examination location by analyzing the ultrasound images of a plurality of temporally consecutive frames such as the optical flow, and determine whether or not the examination location is pressed on the basis of the motion in the examination location.

Further, the blood vessel confirmation unit 63 can determine the presence or absence of the change in the aspect ratio of the cross section of the blood vessel for a predetermined period of time after prompting the user to press the examination location with the ultrasound probe 1, and determine whether or not the examination location is pressed on the basis of the presence or absence of the change in the aspect ratio of the blood vessel in the predetermined period of time. This determination method does not require the above-described pressure sensor and the image analysis such as the detection of the motion.

In this case, it is preferable that the blood vessel confirmation unit 63 causes the display control unit 33 to display, on the monitor 34, the above-described predetermined period of time, that is, the remaining time for determining the presence or absence of the change in the aspect ratio of the cross section of the blood vessel. Thereby, the user can understand the time during which the examination location is pressed with the ultrasound probe 1, in other words, the timing at which pressing the examination location with the ultrasound probe 1 is ended.

Next, the method of using the color Doppler mode will be described.

In this case, the blood vessel confirmation unit 63 prompts the user to incline the ultrasound probe 1 to the peripheral side or the central side at the examination location of the subject. For example, as illustrated in Fig. 14, the blood vessel confirmation unit 63 causes the display control unit 33 to display a message for prompting the user on the monitor 34. In Fig. 14, a schematic diagram representing inclining the ultrasound probe is displayed on the right portion of the ultrasound image, and on the lower side thereof, characters "incline!" are displayed. Similarly, the blood vessel confirmation unit 63 may output sound for prompting the user from the speaker, and may use both the message and the sound.

Subsequently, the blood vessel confirmation unit 63 confirms whether the blood vessel is an artery or a vein on the basis of the direction of the blood flow in the ultrasound image generated while the ultrasound probe 1 is inclined at the examination location in the color Doppler mode. For example, in a case where the ultrasound probe 1 is inclined to the peripheral side, the blood vessel confirmation unit 63 confirms that the blood flow is an artery in a case where the direction of the blood flow is a direction approaching the ultrasound probe 1, and the blood vessel confirmation unit 63 confirms that the blood vessel is a vein in a case where the direction of the blood flow is a direction away from the ultrasound probe 1. On the other hand, in a case where the ultrasound probe 1 is inclined to the central side, the blood vessel confirmation unit 63 confirms results opposite to the results in a case where the ultrasound probe 1 is inclined to the peripheral side.

Since it is only necessary to determine the direction of the blood flow, it is not essential to display the direction of the blood flow in color in the ultrasound image.

Then, after it is confirmed whether the blood vessel is an artery or a vein, the blood vessel confirmation unit 63 ends the color Doppler mode.

Next, the method of using the ultrasound images of the past frames will be described.

In this case, the blood vessel confirmation unit 63 searches for an ultrasound image of a frame in which the probability that the same blood vessel as the blood vessel in the ultrasound image at the time of examination is an artery or the probability that the same blood vessel is a vein is equal to or greater than a fourth threshold value, among the ultrasound images of the past frames stored in the image memory 32. For example, the blood vessel confirmation unit 63 searches for an ultrasound image of a frame with the highest probability that the same blood vessel is an artery or the highest probability that the same blood vessel is a vein, among the ultrasound images of the past frames.

For example, the blood vessel confirmation unit 63 can detect the blood vessel in the ultrasound images of the adjacent preceding and succeeding frames, and determine that the blood vessels are the same in a case where an overlapping ratio of the blood vessels in the ultrasound images of the preceding and succeeding frames is equal to or greater than the fourth threshold value.

Then, the blood vessel confirmation unit 63 confirms that the blood vessel in the ultrasound image at the time of examination is an artery or a vein on the basis of the probability that the same blood vessel in the ultrasound image of the frame searched from the ultrasound images of the past frames is an artery or the probability that the same blood vessel is a vein. That is, as the probability that the same blood vessel in the ultrasound image of the current frame is an artery or the probability that the same blood vessel is a vein, the probability that the same blood vessel in the ultrasound image of the past frame is an artery or the probability that the same blood vessel is a vein is used as is.

The ultrasound diagnostic apparatus determines the type of the blood vessel on the basis of the probability that the blood vessel is an artery and the probability that the blood vessel is a vein as described above, and in a case where the type of the blood vessel cannot be determined, the confirmation processing is executed to confirm whether the blood vessel is an artery or a vein. Therefore, it is possible to prevent the type of the blood vessel from being erroneously determined, and to greatly improve the determination accuracy of the type of the blood vessel. In addition, since the ultrasound diagnostic apparatus executes the confirmation processing only in a case where the type of the blood vessel cannot be determined, it is possible to greatly suppress the deterioration of the usability.

In a case of puncturing the blood vessel using the ultrasound diagnostic apparatus, the user is required to be skilled in scanning the examination location of the subject and interpreting the ultrasound image, and it is not easy for an unskilled person to scan the examination location and interpret the ultrasound image. Accordingly, by determining the region including the blood vessel and the type of the blood vessel, and displaying the determination result on the monitor 34 as described above, it is possible to assist the user in scanning the examination location and interpreting the ultrasound image.

The display control unit 33 can superimpose the graphic including the blood vessel on the ultrasound image to be displayed on the monitor 34 on the basis of at least one of the determination result on whether the blood vessel is an artery or a vein or the confirmation result on whether the blood vessel is an artery or a vein. That is, the graphic including the blood vessel may be superimposed on the ultrasound image to be displayed on the monitor 34 on the basis of both the determination result and the confirmation result.

In this case, for example, the probability calculation unit 61 calculates a score according to the aspect ratio of the cross section of the blood vessel in the ultrasound image generated while the examination location is pressed with the ultrasound probe 1. Next, the probability calculation unit 61 weights and averages the probability that the blood vessel is an artery and the probability that the blood vessel is a vein, with the score of the aspect ratio of the cross section of the blood vessel to calculate a final score that the blood vessel is an artery and a final score that the blood vessel is a vein.

Then, the display control unit 33 superimposes the final score that the blood vessel is an artery and the final score that the blood vessel is a vein on the ultrasound image to be displayed on the monitor 34, instead of the probability that the blood vessel is an artery and the probability that the blood vessel is a vein. Thereby, the user can know the final score that the blood vessel is an artery and the final score that the blood vessel is a vein which are more accurate than the probability that the blood vessel is an artery and the probability that the blood vessel is a vein.

Similarly, the blood vessel determination unit 62 may determine whether the blood vessel is an artery or a vein or whether or not the confirmation processing is required on the basis of the final score that the blood vessel is an artery and the final score that the blood vessel is a vein instead of the probability that the blood vessel is an artery and the probability that the blood vessel is a vein.

The display control unit 33 may display the same graphic for the same blood vessel on the monitor 34 once whether the blood vessel is an artery or a vein has been decided. Thereby, it is possible to greatly reduce the processing for deciding the type of the blood vessel.

Furthermore, the display control unit 33 may display characters "blood vessel" instead of the graphics, on the monitor 34 in a case where whether the blood vessel is an artery or a vein cannot be decided even by the confirmation processing. Thereby, it is possible for the user to know that there is a blood vessel in the region of the graphic.

Among the kinds of confirmation processing such as the method of pressing the examination location, the method of using the color Doppler mode, and the method of using the ultrasound image of the past frame, two or more kinds of confirmation processing may be used in combination.

Here, the method of pressing the examination location requires the user's operation such as pressing the examination location with the ultrasound probe 1, and the method of using the color Doppler mode requires the user's operation such as inclining the ultrasound probe 1 at the examination location. However, the method of using the ultrasound image of the past frame does not require the user's operation. Therefore, it is desirable to execute the confirmation processing step by step such that, in a case where the method of using the ultrasound image of the past frame is executed first, and the type of the blood vessel cannot be confirmed by the execution result, the method of pressing the examination location, the method of using the color Doppler mode, and the like are sequentially executed.

The present invention is not limited to a stationary ultrasound diagnostic apparatus, and can be similarly applied to a portable ultrasound diagnostic apparatus in which an apparatus main body 3 is realized by a laptop terminal device, and a handheld ultrasound diagnostic apparatus in which an apparatus main body 3 is realized by a handheld terminal device such as a smartphone or a tablet personal computer (PC). The ultrasound probe 1 and the apparatus main body 3 may be connected in a wired or wireless manner. Further, the entire image generation unit 31 or only the signal processing unit 16 may be provided on the ultrasound probe 1 side, or provided on the apparatus main body 3 side.

In the apparatus of the present invention, the hardware configurations of the processing units executing various kinds of processing such as the transmission and reception circuit 14, the image generation unit 31, the display control unit 33, the blood vessel processing unit 35, and the apparatus control unit 36 may be dedicated hardware, or may be various processors or computers that execute programs.

The various processors include a central processing unit (CPU) as a general-purpose processor executing software (program) and functioning as various processing units, a programmable logic device (PLD) as a processor of which the circuit configuration can be changed after manufacturing such as a field programmable gate array (FPGA), and a dedicated electric circuit as a processor having a circuit configuration designed exclusively for executing specific processing such as an application specific integrated circuit (ASIC).

One processing unit may be configured by one of the various processors or may be configured by a combination of the same or different kinds of two or more processors, for example, a combination of a plurality of FPGAs or a combination of an FPGA and a CPU). Further, a plurality of processing units may be configured by one of various processors, or two or more of a plurality of processing units may be collectively configured by using one processor.

For example, there is a form where one processor is configured by a combination of one or more CPUs and software as typified by a computer, such as a server and a client, and this processor functions as a plurality of processing units. Further, there is a form where a processor realizing the functions of the entire system including a plurality of processing units by one integrated circuit (IC) chip as typified by a system on chip (SoC) or the like is used.

Furthermore, the hardware configurations of these various processors are more specifically electric circuitry where circuit elements, such as semiconductor elements, are combined.

The method of the present invention can be carried out, for example, by a program for causing a computer to execute each step of the method. Further, a computer-readable recording medium in which this program is recorded can also be provided.

### Explanation of References

1: ultrasound probe
3: apparatus main body
11: transducer array
14: transmission and reception circuit
16: signal processing unit
17: image processing unit
18: DSC
31: image generation unit
32: image memory
33: display control unit
34: monitor
35: blood vessel processing unit
36: apparatus control unit
37: input device
39: processor
51: pulser
52: amplification unit
53: AD conversion unit
54: beam former
61: probability calculation unit
62: blood vessel determination unit
63: blood vessel confirmation unit
71, 72: enclosing line
73, 74: graphic

## Claims

1. An ultrasound diagnostic apparatus comprising:
an ultrasound probe (1);
an image generation unit (31) that generates an ultrasound image from a reception signal obtained by performing transmission and reception of an ultrasound beam with respect to a subject using the ultrasound probe; the ultrasound diagnostic apparatus **characterized by**
a probability calculation unit (61) that calculates each of a probability that a blood vessel in the ultrasound image is an artery and a probability that the blood vessel is a vein by analyzing the ultrasound image;
a blood vessel determination unit (62) that determines whether the blood vessel is the artery or the vein or whether or not confirmation processing for confirming whether the blood vessel is the artery or the vein is required on the basis of the probability that the blood vessel is the artery and the probability that the blood vessel is the vein; and
a blood vessel confirmation unit (62) that executes the confirmation processing in a case where it is determined that the confirmation processing is required, and confirms whether the blood vessel is the artery or the vein on the basis of an execution result of the confirmation processing.

2. The ultrasound diagnostic apparatus according to claim 1,
wherein the probability calculation unit (61) detects a blood vessel region with a predetermined shape, including a blood vessel in the ultrasound image, in a case of calculating a probability of being the artery and a probability of being the vein.

3. The ultrasound diagnostic apparatus according to claim 2,
wherein the blood vessel region is an artery region including the artery or a vein region including the vein, and
the probability calculation unit (61) detects the artery region including the blood vessel in the ultrasound image, calculates a probability that the blood vessel in the artery region is the artery, detects the vein region including the same blood vessel, and calculates a probability that the same blood vessel in the vein region is the vein.

4. The ultrasound diagnostic apparatus according to claim 2,
wherein the probability calculation unit (61) detects a single blood vessel region with the predetermined shape, including a blood vessel in the ultrasound image, and further calculates a probability that the blood vessel in the single blood vessel region is the artery and a probability that the blood vessel in the single blood vessel region is the vein.

5. The ultrasound diagnostic apparatus according to any one of claims 2 to 4,
wherein in a first case in which a probability of being one of the artery and the vein is equal to or greater than a first threshold value and a probability of being the other of the artery and the vein is less than a second threshold value smaller than the first threshold value, the blood vessel determination unit determines that the blood vessel is one of the artery and the vein, and in a second case other than the first case, the blood vessel determination unit determines that the confirmation processing is required.

6. The ultrasound diagnostic apparatus according to claim 1,
wherein the probability calculation unit (61) calculates, for each pixel of the ultrasound image, each of a probability that the pixel is a pixel of the artery, a probability that the pixel is a pixel of the vein, and a probability that the pixel is a pixel of background other than the blood vessel.

7. The ultrasound diagnostic apparatus according to claim 6,
wherein the blood vessel determination unit (61) decides, for each pixel of the ultrasound image, whether the pixel is the pixel of the artery, the pixel is the pixel of the vein, or the pixel is the pixel of the background on the basis of the probability that the pixel is the pixel of the artery, the probability that the pixel is the pixel of the vein, and the probability that the pixel is the pixel of the background, decides a blood vessel region including the blood vessel on the basis of the pixel of the artery and the pixel of the vein, determines that the blood vessel is one of the artery and the vein in a case where a ratio of one of an area of the pixel of the artery and an area of the pixel of the vein to an area of the blood vessel in the blood vessel region is equal to or greater than a third threshold value, and determines that the confirmation processing is required in a case where the ratio is less than the third threshold value.

8. The ultrasound diagnostic apparatus according to any one of claims 1 to 7,
wherein the blood vessel confirmation unit (62) prompts a user to press an examination location of the subject with the ultrasound probe, calculates an aspect ratio of a cross section of the blood vessel on the basis of an ultrasound image generated while the examination location is pressed with the ultrasound probe, and confirms whether the blood vessel is the artery or the vein on the basis of the aspect ratio of the cross section of the blood vessel.

9. The ultrasound diagnostic apparatus according to claim 8,
wherein the ultrasound probe (1) has a pressure sensor, and
the blood vessel confirmation unit determines whether or not the examination location is pressed on the basis of a pressure detected by the pressure sensor.

10. The ultrasound diagnostic apparatus according to claim 8,
wherein the blood vessel confirmation unit (62) detects motion in the examination location by analyzing the ultrasound images of a plurality of frames, and determines whether or not the examination location is pressed on the basis of the motion in the examination location.

11. The ultrasound diagnostic apparatus according to claim 8,
wherein the blood vessel confirmation unit (62) determines presence or absence of a change in the aspect ratio of the cross section of the blood vessel only for a predetermined period of time after prompting the user to press the examination location with the ultrasound probe, and determines whether or not the examination location is pressed on the basis of the presence or absence of the change in the aspect ratio of the blood vessel in the predetermined period of time.

12. The ultrasound diagnostic apparatus according to any one of claims 1 to 11,
wherein the blood vessel confirmation unit (62) prompts a user to incline the ultrasound probe at an examination location of the subject, and confirms whether the blood vessel is the artery or the vein on the basis of a direction of a blood flow in an ultrasound image generated while the ultrasound probe is inclined at the examination location in a color Doppler mode.

13. The ultrasound diagnostic apparatus according to any one of claims 1 to 12,
wherein the blood vessel confirmation unit (62) searches for an ultrasound image of a frame in which a probability that the same blood vessel is the artery or a probability that the same blood vessel is the vein is equal to or greater than a fourth threshold value among ultrasound images of past frames, and confirms whether the blood vessel is the artery or the vein on the basis of the probability that the same blood vessel in the ultrasound image of the searched frame is the artery or the probability that the same blood vessel is the vein.

14. The ultrasound diagnostic apparatus according to claim 13,
wherein the blood vessel confirmation unit (62) detects the blood vessel in ultrasound images of adjacent preceding and succeeding frames, and determines that the blood vessels are the same in a case where an overlapping ratio of the blood vessels in the ultrasound images of the preceding and succeeding frames is equal to or greater than the fourth threshold value.

15. The ultrasound diagnostic apparatus according to any one of claims 1 to 14, further comprising:
a monitor (34); and
a display control unit (33) that superimposes a graphic (73, 74) including the blood vessel on the ultrasound image to be displayed on the monitor on the basis of at least one of a determination result on whether the blood vessel is the artery or the vein or a confirmation result on whether the blood vessel is the artery or the vein.

16. The ultrasound diagnostic apparatus according to claim 15,
wherein the display control unit displays each of the probability that the blood vessel is the artery and the probability that the blood vessel is the vein on the monitor in addition to the graphic.

17. The ultrasound diagnostic apparatus according to claim 15 or 16,
wherein the display control unit displays the same graphic for the same blood vessel on the monitor once whether the blood vessel is the artery or the vein has been decided.

18. The ultrasound diagnostic apparatus according to any one of claims 15 to 17,
wherein the display control unit displays characters, blood vessel, on the monitor in addition to the graphic in a case where deciding whether the blood vessel is the artery or the vein is not possible.

19. A control method of an ultrasound diagnostic apparatus, the control method comprising:
generating an ultrasound image from a reception signal obtained by performing transmission and reception of an ultrasound beam with respect to a subject using an ultrasound probe; the control method **characterized by**
calculating a probability that a blood vessel in the ultrasound image is an artery and a probability that the blood vessel is a vein by analyzing the ultrasound image;
determining whether the blood vessel is the artery or the vein or whether or not confirmation processing for confirming whether the blood vessel is the artery or the vein is required on the basis of the probability that the blood vessel is the artery and the probability that the blood vessel is the vein; and
executing the confirmation processing in a case where it is determined that the confirmation processing is required, and confirming whether the blood vessel is the artery or the vein on the basis of an execution result of the confirmation processing.

## Patentansprüche

1. Ultraschalldiagnosevorrichtung, umfassend:
eine Ultraschallsonde (1);
eine Bilderzeugungseinheit (31), die ein Ultraschallbild aus einem Empfangssignal erzeugt, das durch Durchführen von Transmission Empfang eines Ultraschallstrahls in Bezug auf ein Motiv unter Verwendung der Ultraschallsonde erhalten wird;
wobei die Ultraschalldiagnosevorrichtung **gekennzeichnet ist: durch**
eine Wahrscheinlichkeitsberechnungseinheit (61), die **durch** Analysieren des Ultraschallbildes jeweils eine Wahrscheinlichkeit, dass ein Blutgefäß in dem Ultraschallbild eine Arterie ist, und eine Wahrscheinlichkeit, dass das Blutgefäß eine Vene ist, berechnet;
eine Blutgefäß-Bestimmungseinheit (62), die auf der Grundlage der Wahrscheinlichkeit, dass das Blutgefäß die Arterie ist, und der Wahrscheinlichkeit, dass das Blutgefäß die Vene ist, bestimmt, ob das Blutgefäß die Arterie oder die Vene ist oder ob ein Überprüfungsverfahren zum Überprüfen, ob das Blutgefäß die Arterie oder die Vene ist, erforderlich ist oder nicht; und
eine Blutgefäß-Überprüfungseinheit (62), die in einem Fall, in dem bestimmt wird, dass Überprüfungsverarbeitung erforderlich ist, die Überprüfungsverarbeitung ausführt und auf der Grundlage eines Ausführungsergebnisses der Überprüfungsverarbeitung überprüft, ob das Blutgefäß die Arterie oder die Vene ist.

2. Ultraschalldiagnosevorrichtung nach Anspruch 1,
wobei die Wahrscheinlichkeitsberechnungseinheit (61) einen Blutgefäßbereich mit einer vorbestimmten Form einschließlich eines Blutgefäßes in dem Ultraschallbild in einem Fall des Berechnens einer Wahrscheinlichkeit, dass es die Arterie ist, und einer Wahrscheinlichkeit, dass es die Vene ist, detektiert.

3. Ultraschalldiagnosevorrichtung nach Anspruch 2,
wobei der Blutgefäßbereich ein Arterienbereich ist, der die Arterie umfasst, oder ein Venenbereich, der die Vene umfasst, und
die Wahrscheinlichkeitsberechnungseinheit (61) den Arterienbereich, der das Blutgefäß umfasst, in dem Ultraschallbild detektiert, eine Wahrscheinlichkeit, dass das Blutgefäß in dem Arterienbereich die Arterie ist, berechnet, den Venenbereich, der dasselbe Blutgefäß umfasst, detektiert und eine Wahrscheinlichkeit, dass dasselbe Blutgefäß in dem Venenbereich die Vene ist, berechnet.

4. Ultraschalldiagnosevorrichtung nach Anspruch 2,
wobei die Wahrscheinlichkeitsberechnungseinheit (61) einen einzelnen Blutgefäßbereich mit der vorgegebenen Form einschließlich eines Blutgefäßes in dem Ultraschallbild detektiert und ferner eine Wahrscheinlichkeit, dass das Blutgefäß in dem einzelnen Blutgefäßbereich die Arterie ist, und eine Wahrscheinlichkeit, dass das Blutgefäß in dem einzelnen Blutgefäßbereich die Vene ist, berechnet.

5. Ultraschalldiagnosevorrichtung nach einem der Ansprüche 2 bis 4,
wobei in einem ersten Fall, in dem eine Wahrscheinlichkeit, dass eine von der Arterie und der Vene gleich oder größer als ein erster Schwellenwert ist, und eine Wahrscheinlichkeit, dass die andere von der Arterie und der Vene kleiner als ein zweiter Schwellenwert ist, der kleiner als der erste Schwellenwert ist, die Blutgefäßbestimmungseinheit bestimmt, dass das Blutgefäß eine von der Arterie und der Vene ist, und in einem zweiten Fall, der von dem ersten Fall verschieden ist, die Blutgefäßbestimmungseinheit bestimmt, dass die Überprüfungsverarbeitung erforderlich ist.

6. Ultraschalldiagnosevorrichtung nach Anspruch 1,
wobei die Wahrscheinlichkeitsberechnungseinheit (61) für jedes Pixel des Ultraschallbildes jeweils eine Wahrscheinlichkeit, dass das Pixel ein Pixel der Arterie ist, eine Wahrscheinlichkeit, dass das Pixel ein Pixel der Vene ist, und eine Wahrscheinlichkeit, dass das Pixel ein Pixel eines Hintergrunds ist, der von dem Blutgefäß verschieden ist, berechnet.

7. Ultraschalldiagnosevorrichtung nach Anspruch 6,
wobei die Blutgefäßbestimmungseinheit (61) für jedes Pixel des Ultraschallbildes auf der Grundlage der Wahrscheinlichkeit, dass das Pixel das Pixel der Arterie ist, der Wahrscheinlichkeit, dass das Pixel das Pixel der Vene ist, und der Wahrscheinlichkeit, dass das Pixel das Pixel des Hintergrunds ist, bestimmt, ob das Pixel das Pixel der Arterie ist, das Pixel das Pixel der Vene ist oder das Pixel das Pixel des Hintergrunds ist, einen Blutgefäßbereich, der das Blutgefäß umfasst, auf der Grundlage des Pixels der Arterie und des Pixels der Vene bestimmt, bestimmt, dass das Blutgefäß eine von der Arterie und der Vene ist, in einem Fall, in dem ein Verhältnis einer von einer Fläche des Pixels der Arterie und einer Fläche des Pixels der Vene zu einer Fläche des Blutgefäßes in dem Blutgefäßbereich gleich oder größer als ein dritter Schwellenwert ist, und bestimmt, dass die Überprüfungsverarbeitung erforderlich ist, in einem Fall, in dem das Verhältnis kleiner als der dritte Schwellenwert ist.

8. Ultraschalldiagnosevorrichtung nach einem der Ansprüche 1 bis 7,
wobei die Blutgefäß-Überprüfungseinheit (62) einen Benutzer auffordert, eine Untersuchungsstelle der Untersuchungsperson mit der Ultraschallsonde zu drücken, ein Seitenverhältnis eines Querschnitts des Blutgefäßes auf der Grundlage eines Ultraschallbildes, das erzeugt wird, während die Untersuchungsstelle mit der Ultraschallsonde gedrückt wird, berechnet und auf der Grundlage des Seitenverhältnisses des Querschnitts des Blutgefäßes überprüft, ob das Blutgefäß die Arterie oder die Vene ist.

9. Ultraschalldiagnosevorrichtung nach Anspruch 8,
wobei die Ultraschallsonde (1) einen Drucksensor aufweist, und
die Blutgefäß-Überprüfungseinheit auf der Grundlage eines von dem Drucksensor detektierten Drucks bestimmt, ob die Untersuchungsstelle gedrückt wird oder nicht.

10. Ultraschalldiagnosevorrichtung nach Anspruch 8,
wobei die Blutgefäß-Überprüfungseinheit (62) Bewegung an der Untersuchungsstelle durch Analysieren der Ultraschallbilder von mehreren Einzelbildern detektiert und auf der Grundlage der Bewegung an der Untersuchungsstelle bestimmt, ob die Untersuchungsstelle gedrückt wird oder nicht.

11. Ultraschalldiagnosevorrichtung nach Anspruch 8,
wobei die Blutgefäß-Überprüfungseinheit (62) Vorhandensein oder Nichtvorhandensein einer Änderung des Seitenverhältnisses des Querschnitts des Blutgefäßes nur für einen vorbestimmten Zeitraum bestimmt, nachdem der Benutzer aufgefordert wurde, die Untersuchungsstelle mit der Ultraschallsonde zu drücken, und auf der Grundlage des Vorhandenseins oder Nichtvorhandenseins der Änderung des Seitenverhältnisses des Blutgefäßes in dem vorbestimmten Zeitraum bestimmt, ob die Untersuchungsstelle gedrückt wird oder nicht.

12. Ultraschalldiagnosevorrichtung nach einem der Ansprüche 1 bis 11,
wobei die Blutgefäß-Überprüfungseinheit (62) den Benutzer auffordert, die Ultraschallsonde an einer Untersuchungsstelle der Untersuchungsperson zu neigen, und auf der Grundlage einer Richtung eines Blutflusses in einem Ultraschallbild, das in einem Farb-Doppler-Modus erzeugt wird, während die Ultraschallsonde an der Untersuchungsstelle geneigt wird, überprüft, ob das Blutgefäß die Arterie oder die Vene ist.

13. Ultraschalldiagnosevorrichtung nach einem der Ansprüche 1 bis 12,
wobei die Blutgefäß-Überprüfungseinheit (62) nach einem Ultraschallbild eines Einzelbildes, in dem eine Wahrscheinlichkeit, dass dasselbe Blutgefäß die Arterie ist, oder eine Wahrscheinlichkeit, dass dasselbe Blutgefäß die Vene ist, gleich oder größer als ein vierter Schwellenwert ist, unter den Ultraschallbildern vergangener Einzelbilder sucht und auf der Grundlage der Wahrscheinlichkeit, dass dasselbe Blutgefäß in dem Ultraschallbild des gesuchten Einzelbildes die Arterie ist, oder der Wahrscheinlichkeit, dass dasselbe Blutgefäß die Vene ist, überprüft, ob das Blutgefäß die Arterie oder die Vene ist.

14. Ultraschalldiagnosevorrichtung nach Anspruch 13,
wobei die Blutgefäß-Überprüfungseinheit (62) das Blutgefäß in Ultraschallbildern benachbarter vorhergehender und nachfolgender Einzelbilder detektiert und in einem Fall, in dem ein Überlappungsverhältnis der Blutgefäße in den Ultraschallbildern der vorhergehenden und nachfolgenden Einzelbilder gleich oder größer als der vierte Schwellenwert ist, bestimmt, dass die Blutgefäße gleich sind.

15. Ultraschalldiagnosevorrichtung nach einem der Ansprüche 1 bis 14, ferner umfassend:
einen Monitor (34); und
eine Anzeigesteuereinheit (33), die eine Grafik (73, 74), die das Blutgefäß enthält, dem Ultraschallbild, das auf dem Monitor anzuzeigen ist, auf der Grundlage mindestens eines von einem einem Bestimmungsergebnis darüber, ob das Blutgefäß die Arterie oder die Vene ist, und einem Überprüfungsergebnis darüber, ob das Blutgefäß die Arterie oder die Vene ist, überlagert.

16. Ultraschalldiagnosevorrichtung nach Anspruch 15,
wobei die Anzeigesteuereinheit zusätzlich zu der Grafik jeweils die Wahrscheinlichkeit, dass das Blutgefäß die Arterie ist, und die Wahrscheinlichkeit, dass das Blutgefäß die Vene ist, auf dem Monitor anzeigt.

17. Ultraschalldiagnosevorrichtung nach Anspruch 15 oder 16,
wobei die Anzeigesteuereinheit dieselbe Grafik für dasselbe Blutgefäß auf dem Monitor anzeigt, sobald bestimmt wurde, ob das Blutgefäß die Arterie oder die Vene ist.

18. Ultraschalldiagnosevorrichtung nach einem der Ansprüche 15 bis 17,
wobei die Anzeigesteuereinheit zusätzlich zu der Grafik Zeichen und Blutgefäß auf dem Monitor in einem Fall, in dem es nicht möglich ist zu bestimmen, ob das Blutgefäß die Arterie oder die Vene ist, anzeigt.

19. Steuerverfahren einer Ultraschalldiagnosevorrichtung, wobei das Steuerverfahren umfasst:
Erzeugen eines Ultraschallbildes aus einem Empfangssignal, das durch Durchführen von Transmission Empfang eines Ultraschallstrahls in Bezug auf ein Motiv unter Verwendung einer Ultraschallsonde erhalten wird;
wobei das Steuerverfahren, **gekennzeichnet ist: durch**
Berechnen, **durch** Analysieren des Ultraschallbildes, einer Wahrscheinlichkeit, dass ein Blutgefäß in dem Ultraschallbild eine Arterie ist, und einer Wahrscheinlichkeit, dass das Blutgefäß eine Vene ist;
Bestimmen auf der Grundlage der Wahrscheinlichkeit, dass das Blutgefäß die Arterie ist, und der Wahrscheinlichkeit, dass das Blutgefäß die Vene ist, ob das Blutgefäß die Arterie oder die Vene ist oder ob ein Überprüfungsverfahren zum Überprüfen, ob das Blutgefäß die Arterie oder die Vene ist, erforderlich ist oder nicht; und
Ausführen, in einem Fall, in dem bestimmt wird, dass Überprüfungsverarbeitung erforderlich ist, der Überprüfungsverarbeitung und Überprüfen auf der Grundlage eines Ausführungsergebnisses der Überprüfungsverarbeitung, ob das Blutgefäß die Arterie oder die Vene ist.

## Revendications

1. Appareil de diagnostic par ultrasons comprenant :
une sonde ultrasonore (1) ;
une unité de génération d'images (31) qui génère une image ultrasonore à partir d'un signal de réception obtenu en effectuant transmission et réception d'un faisceau ultrasonore par rapport à un sujet en utilisant la sonde ultrasonore ;
l'appareil de diagnostic par ultrasons **caractérisé par**
une unité de calcul de probabilité (61) qui calcule chacune de une probabilité qu'un vaisseau sanguin dans l'image ultrasonore soit une artère et une probabilité que le vaisseau sanguin soit une veine en analysant l'image ultrasonore ;
une unité de détermination de vaisseau sanguin (62) qui détermine si le vaisseau sanguin est une artère ou une veine ou si oui ou non un traitement de confirmation pour vérifier si le vaisseau sanguin est une artère ou une veine est requis sur la base de la probabilité que le vaisseau sanguin soit une artère et de la probabilité que le vaisseau sanguin soit une veine ; et
une unité de confirmation de vaisseau sanguin (62) qui exécute le traitement de confirmation dans un cas où il est déterminé que le traitement de confirmation est requis, et confirme si le vaisseau sanguin est une artère ou une veine sur la base d'un résultat d'exécution du traitement de confirmation.

2. Appareil de diagnostic par ultrasons selon la revendication 1,
dans lequel l'unité de calcul de probabilité (61) détecte une région de vaisseau sanguin avec une forme prédéterminée, incluant un vaisseau sanguin dans l'image ultrasonore, dans un cas du calcul d'une probabilité d'être l'artère et d'une probabilité d'être la veine.

3. Appareil de diagnostic par ultrasons selon la revendication 2,
dans lequel la région du vaisseau sanguin est une région artérielle incluant l'artère ou une région veineuse incluant la veine, et
l'unité de calcul de probabilité (61) détecte la région artérielle incluant le vaisseau sanguin dans l'image ultrasonore, calcule une probabilité que le vaisseau sanguin dans la région artérielle soit l'artère, détecte la région veineuse incluant le même vaisseau sanguin, et calcule une probabilité que le même vaisseau sanguin dans la région veineuse soit la veine.

4. Appareil de diagnostic par ultrasons selon la revendication 2,
dans lequel l'unité de calcul de probabilité (61) détecte une seule région de vaisseau sanguin avec la forme prédéterminée, incluant un vaisseau sanguin dans l'image ultrasonore, et calcule en outre une probabilité que le vaisseau sanguin dans la seule région de vaisseau sanguin soit l'artère et une probabilité que le vaisseau sanguin dans la seule région de vaisseau sanguin soit la veine.

5. Appareil de diagnostic par ultrasons selon l'une quelconque des revendications 2 à 4, dans lequel dans un premier cas où une probabilité d'être une de l'artère et de la veine est égale ou supérieure à une première valeur seuil et une probabilité d'être l'autre de l'artère et de la veine est inférieure à une deuxième valeur seuil plus petite que la première valeur seuil, l'unité de détermination de vaisseau sanguin détermine que le vaisseau sanguin est une de l'artère et de la veine, et dans un deuxième cas autre que le premier cas, l'unité de détermination de vaisseau sanguin détermine que le traitement de confirmation est requis.

6. Appareil de diagnostic par ultrasons selon la revendication 1,
dans lequel l'unité de calcul de probabilité (61) calcule, pour chaque pixel de l'image ultrasonore, chacune d'une probabilité que le pixel soit un pixel de l'artère, une probabilité que le pixel soit un pixel de la veine et une probabilité que le pixel soit un pixel de fond autre que le vaisseau sanguin.

7. Appareil de diagnostic par ultrasons selon la revendication 6,
dans lequel l'unité de détermination de vaisseau sanguin (61) décide, pour chaque pixel de l'image ultrasonore, si le pixel est le pixel de l'artère, le pixel est le pixel de la veine ou le pixel est le pixel de fond sur la base de la probabilité que le pixel soit le pixel de l'artère, la probabilité que le pixel soit le pixel de la veine et la probabilité que le pixel soit le pixel de fond, décide d'une région de vaisseau sanguin incluant le vaisseau sanguin sur la base du pixel de l'artère et du pixel de la veine, détermine que le vaisseau sanguin est une de l'artère et de la veine dans un cas où un rapport d'une d'une surface du pixel de l'artère et d'une surface du pixel de la veine à une surface du vaisseau sanguin dans la région du vaisseau sanguin est égal ou supérieur à une troisième valeur seuil, et détermine que le traitement de confirmation est requis dans un cas où le rapport est inférieur à la troisième valeur seuil.

8. Appareil de diagnostic par ultrasons selon l'une quelconque des revendications 1 à 7, dans lequel l'unité de confirmation de vaisseau sanguin (62) demande un utilisateur d'appuyer sur un emplacement d'examen du sujet avec la sonde ultrasonore, calcule un rapport d'aspect d'une section transversale du vaisseau sanguin sur la base d'une image ultrasonore générée pendant que l'emplacement d'examen est pressé avec la sonde ultrasonore, et confirme si le vaisseau sanguin est l'artère ou la veine sur la base du rapport d'aspect de la section transversale du vaisseau sanguin.

9. Appareil de diagnostic par ultrasons selon la revendication 8,
dans lequel la sonde ultrasonore (1) comporte un capteur de pression, et
l'unité de confirmation de vaisseau sanguin détermine si oui ou non l'emplacement d'examen est pressé sur la base d'une pression détectée par le capteur de pression.

10. Appareil de diagnostic par ultrasons selon la revendication 8,
dans lequel l'unité de confirmation de vaisseau sanguin (62) détecte mouvement dans l'emplacement d'examen en analysant les images ultrasonores d'une pluralité de trames, et détermine si oui ou non l'emplacement d'examen est pressé sur la base du mouvement dans l'emplacement d'examen.

11. Appareil de diagnostic par ultrasons selon la revendication 8,
dans lequel l'unité de confirmation de vaisseau sanguin (62) détermine présence ou absence d'un changement dans le rapport d'aspect de la section transversale du vaisseau sanguin uniquement pendant une période de temps prédéterminée après avoir demandé l'utilisateur à appuyer sur l'emplacement d'examen avec la sonde ultrasonore, et détermine si oui ou non l'emplacement d'examen est pressé sur la base de la présence ou de l'absence du changement dans le rapport d'aspect du vaisseau sanguin pendant la période de temps prédéterminée.

12. Appareil de diagnostic par ultrasons selon l'une quelconque des revendications 1 à 11, dans lequel l'unité de confirmation de vaisseau sanguin (62) demande un utilisateur d'incliner la sonde ultrasonore à un emplacement d'examen du sujet, et confirme si le vaisseau sanguin est l'artère ou la veine sur la base d'une direction d'un flux sanguin dans une image ultrasonore générée pendant que la sonde ultrasonore est inclinée à l'emplacement d'examen en mode Doppler couleur.

13. Appareil de diagnostic par ultrasons selon l'une quelconque des revendications 1 à 12, dans lequel l'unité de confirmation de vaisseau sanguin (62) recherche une image ultrasonore d'une trame dans laquelle une probabilité que le même vaisseau sanguin soit l'artère ou une probabilité que le même vaisseau sanguin soit la veine est égale ou supérieure à une quatrième valeur seuil parmi les images ultrasonores des trames passées, et confirme si le vaisseau sanguin est l'artère ou la veine sur la base de la probabilité que le même vaisseau sanguin dans l'image ultrasonore de la trame recherchée soit l'artère ou la probabilité que le même vaisseau sanguin soit la veine.

14. Appareil de diagnostic par ultrasons selon la revendication 13,
dans lequel l'unité de confirmation de vaisseau sanguin (62) détecte le vaisseau sanguin dans les images ultrasonores des cadres adjacents précédents et suivants, et détermine que les vaisseaux sanguins sont identiques dans un cas où un rapport de chevauchement des vaisseaux sanguins dans les images ultrasonores des cadres précédents et suivants est égal ou supérieur à la quatrième valeur seuil.

15. Appareil de diagnostic par ultrasons selon l'une quelconque des revendications 1 à 14, comprenant en outre :
un moniteur (34) ; et
une unité de contrôle d'affichage (33) qui superpose un graphique (73, 74) incluant le vaisseau sanguin sur l'image ultrasonore à afficher sur le moniteur sur la base d'au moins un d'un résultat de détermination concernant si le vaisseau sanguin est l'artère ou la veine ou d'un résultat de confirmation concernant si le vaisseau sanguin est l'artère ou la veine.

16. Appareil de diagnostic par ultrasons selon la revendication 15,
dans lequel l'unité de contrôle d'affichage affiche chacune de la probabilité que le vaisseau sanguin soit l'artère et la probabilité que le vaisseau sanguin soit la veine sur le moniteur en plus du graphique.

17. Appareil de diagnostic par ultrasons selon la revendication 15 ou la revendication 16, dans lequel l'unité de contrôle d'affichage affiche le même graphique pour le même vaisseau sanguin sur le moniteur une fois qu'il a été décidé si le vaisseau sanguin est l'artère ou la veine.

18. Appareil de diagnostic par ultrasons selon l'une quelconque des revendications 15 à 17,
dans lequel l'unité de contrôle d'affichage affiche des caractères, vaisseau sanguin, sur le moniteur en plus du graphique dans un cas où il n'est pas possible de décider si le vaisseau sanguin est l'artère ou la veine.

19. Procédé de contrôle d'un appareil de diagnostic par ultrasons, le procédé de contrôle comprenant :
générer une image ultrasonore à partir d'un signal de réception obtenu en effectuant transmission et réception d'un faisceau ultrasonore par rapport à un sujet en utilisant une sonde ultrasonore ;
le procédé de contrôle **caractérisé par**
calculer une probabilité qu'un vaisseau sanguin dans l'image ultrasonore soit une artère et une probabilité que le vaisseau sanguin soit une veine en analysant l'image ultrasonore ;
déterminer si le vaisseau sanguin est l'artère ou la veine ou si oui ou non un traitement de confirmation pour vérifier si le vaisseau sanguin est l'artère ou la veine est requis sur la base de la probabilité que le vaisseau sanguin soit l'artère et de la probabilité que le vaisseau sanguin soit la veine ; et
exécuter le traitement de confirmation dans un cas où il est déterminé que le traitement de confirmation est requis, et confirmer si le vaisseau sanguin est l'artère ou la veine sur la base d'un résultat d'exécution du traitement de confirmation.
